(19) Europäisches Patentamt / European Patent Office / Office européen des brevets

(11) **EP 1 206 929 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**28.11.2018  Patentblatt 2018/48**

(45) Hinweis auf die Patenterteilung:
**09.06.2010  Patentblatt 2010/23**

(21) Anmeldenummer: **01123821.9**

(22) Anmeldetag: **05.10.2001**

(51) Int Cl.:
*A61Q 5/12* (2006.01)   *A61K 8/04* (2006.01)
*A61K 8/34* (2006.01)   *A61K 8/41* (2006.01)
*A61K 8/44* (2006.01)   *A61K 8/81* (2006.01)
*A61Q 5/10* (2006.01)

(54) **Verwendung von auf den isoelektrischen Punkt von Haaren eingestellten Zubereitungen zur Konditionierung von oxidativ gefärbten Haaren**

Use of preparations set up at the isoelectric point of hair for conditioning hair under oxidative dyeing

Utilisation de préparations ajustées au point isoélectrique des cheveux pour le conditionnement de cheveux en teinture oxydante

(84) Benannte Vertragsstaaten:
**AT BE CH CY DE DK ES FI FR GB GR IE IT LI LU MC NL PT SE TR**

(30) Priorität: **16.11.2000  DE 10056909**

(43) Veröffentlichungstag der Anmeldung:
**22.05.2002  Patentblatt 2002/21**

(73) Patentinhaber: **The Procter & Gamble Company Cincinnati, OH 45202 (US)**

(72) Erfinder:
• **Hehner, Ursula**
  **64395 Brensbach (DE)**
• **Deutz, Herbert**
  **64347 Griesheim (DE)**
• **Braun, Petra**
  **64839 Münster (DE)**
• **Flemming, Ernst**
  **63150 Heusenstamm (DE)**

(74) Vertreter: **Herzog, Fiesser & Partner Patentanwälte PartG mbB Isartorplatz 1 80331 München (DE)**

(56) Entgegenhaltungen:
**US-A- 4 240 450    US-A- 5 114 428
US-A- 5 580 494**

EP 1 206 929 B2

**Beschreibung**

Gegenstand der Erfindung ist ein schaumbildendes Aerosol-Produkt gemäß Anspruch 1 und ein Verfahren zur Konditionierung und oxidativen Färbung von Haaren gemäß Anspruch 2.

Bei Haarfärbungen unterscheidet man zwischen temporären, semi-permanenten und permanenten Haarfärbungen. Für temporäre Färbungen, den sogenannten Tönungen, werden in der Regel synthetische direktziehende oder natürliche Farbstoffe verwendet, welche das Haar färben, indem sie auf das Haar aufziehen. Für permanente und semi-permanente Färbungen werden in der Regel Oxidationsfarben verwendet, bei denen die Haarfärbung auf der oxidativen Entwicklung der Farbstoffe aus Farbstoffvorprodukten im Inneren des Haares beruht. Der Vorteil der direktziehenden Farbstoffe gegenüber den Oxidationsfarben besteht in einer schonenderen Behandlung des Haares, da keine oxidative Schädigung des Haares eintritt. Der Nachteil der direktziehenden Farbstoffe besteht in der geringeren Haltbarkeit der Färbungen gegenüber Auswaschen. Die direktziehenden Farbstoffe werden durch das Waschen der Haare schnell wieder entfernt, da sie im Gegensatz zu oxidativen Haarfarben nicht den Cortex des Haares färben, sondern sich nur an der Schuppenschicht anlagern. Dies bewirkt bereits nach wenigen Haarwäschen eine starke Farbveränderung oder eine starke Abschwächung der Grauabdeckung. Aber auch oxidative Haarfärbungen sind insbesondere im Rottonbereich gegenüber Haarreinigungsmitteln nicht völlig resistent gegen mehr oder weniger starkes Auswaschen. Bei oxidativ gefärbten Haaren ist eine der Färbung nachfolgende Behandlung mit haarkonditionierend wirkenden Mitteln zu empfehlen, um den oxidativen Schädigungen des Haares entgegenzuwirken. Herkömmliche haarkonditionierende Präparate sind auf der Basis von Fettalkoholen und kationischen Tensiden enthaltenden wässrigen Emulsionen aufgebaut. Die DE 197 35 865 beschreibt die Verwendung eines Mittels enthaltend eine quaternäre Ammoniumverbindung, einen Grünen Tee-Extrakt und ein kationisches Polymer zur Verbesserung der Farbstabilität von gefärbten Haaren, wobei oxidative Haarfärbungen und deren Stabilität gegenüber Auswaschen mit Haarreinigungsmitteln nicht erwähnt werden und der pH-Wert als nicht kritisch bezeichnet wird. Die Anwendung derartiger Produkte als Nachbehandlungsmittel nach oxidativen Haarfärbungen bewirkt zwar eine gewisse Konditionierung der Haare, die Farbstabilität gegenüber Auswaschen bzw. Verblassen der Färbung bei mehrmaliger Behandlung mit Haarreinigungsmitteln ist aber nicht zufriedenstellend.

Es stellte sich somit die Aufgabe, oxidativ gefärbte Haare zu konditionieren und gleichzeitig die Haltbarkeit der oxidativen Haarfärbungen gegenüber dem Waschvorgang zu verlängern. Gelöst wurde diese Aufgabe durch die Verwendung einer auf den isoelektrischen Punkt von menschlichen Haaren eingestellten wässrigen haarkonditionierenden Zubereitung. Bei einer Nachbehandlung von oxidativ gefärbten Haaren mit einer Zubereitung, die auf den isoelektrischen Punkt von menschlichen Haaren eingestellt war und nur ein haarkonditionierendes kationisches Polymer enthielt, wurde gefunden, dass das gefärbte Haar, welches mit der erfindungsgemäßen Zubereitung behandelt wurde, gleichzeitig eine gute Konditionierung und eine verbesserte Farbstabilität gegenüber dem durch Behandlung mit herkömmlichen Konditioniermitteln und anschließendem Shampoonieren erzeugten Farbauswascheffekt aufwies.

Gegenstand der Erfindung ist ein schaumbildendes Aerosol-Produkt enthaltend eine auf den isoelektrischen Punkt von menschlichen Haaren eingestellte wässrige Lösung gemäß Anspruch 1.

Gegenstand der Erfindung ist auch ein Verfahren zur Konditionierung und oxidativen Färbung von Haaren, gemäß Anspruch 2.

Haare bestehen aus Proteinen. Proteine sind charkaterisiert u.a. durch einen isoionischen Punkt und durch einen isoelektrischen Punkt (C.R.Robbins, Chemical and Physical Behavior of Human Hair, 3. Auflage, S. 204-206, 267). Der isoionische Punkt ist derjenige pH-Wert, bei dem die Anzahl sämtlicher positiver Ladungen eines Proteins gleich groß ist wie die Anzahl sämtlicher negativer Ladungen. Der isoionische Punkt von Haaren liegt bei etwa pH 6,0. Der ioselektrische Punkt ist eine Oberflächeneigenschaft von festen Proteinen und ist derjenige pH-Wert, bei welchem die Anzahl der positiven und negativen Ladungen an der Oberfläche des Proteins gleich groß ist. Der isoelektrische Punkt von menschlichem Haar liegt normalerweise bei ca. pH 3,7, wobei der genaue Wert eines individuellen Haares geringfügig von diesem Wert abweichen kann. Erfindungsgemäß auf den isoelektrischen Punkt eingestellte Zubereitungen sind solche, die auf einen pH-Wert im Bereich von 3,6 bis 3,8 eingestellt sind.

Vergleichsversuche zeigen, dass auf pH 3,7 oder pH 3,8 eingestellte haarkonditionierende Zusammensetzungen zu einer besseren Farbstabilität gegenüber Auswaschen führen als wenn diese Zusammensetzungen auf pH 4,5 eingestellt sind. Eine besonders gute Farbstabilität wird durch den gleichzeitigen Einsatz von kationischen Polymeren erreicht. Das erfindungsgemäße kationisches Polymer ist Dimethyldiallylammoniumchlorid/Acrylamid Copolymer (Polyquatemium-7, Merquat® 550 L).

Die Applikationsform ist ein Aerosolschaum. Das erfindungsgemäße Aerosol-Schaumprodukt ist in Anspruch 1 definiert.

Das Polymere mit kationischen Gruppen wird vorzugsweise in einer Menge von 0,001 bis 2,5 Gew.%, besonders bevorzugt von 0,002 bis 1,5 Gew.% eingesetzt. Als kationische Polymere werden solche Polymere verstanden, welche mindestens eine kationische oder durch Protonierung kationisierbare Gruppe enthalten. Kationische Gruppen sind beispielsweise quaternäre Amingruppen, kationisierbare Gruppen sind beispielsweise primäre, sekundäre oder tertiäre Amingruppen. Die kationischen Polymere können Homo- oder Copolymere sein, wobei die kationischen bzw. kationisierbaren Gruppen entweder in der Polymerkette oder vorzugsweise als Substituent an einem oder mehreren der Monomeren enthalten sind.

Monomere kationischer Polymere, welche kationisierbare Gruppen aufweisen, sind ungesättigte, radikalisch polymerisierbare Verbindungen, welche mindestens eine neutralisierte oder nicht neutralisierte basische Gruppe tragen. Als basische Gruppen kommen insbesondere primäre, sekundäre oder tertiäre Amine in Betracht, wobei das Aminstickstoffatom auch Teil eines Ringes sein kann. Beispiele für derartige Monomere sind Mono- und Dialkylaminoalkylacrylate oder -methacrylate. Die Alkylgruppen dieser Monomere sind vorzugsweise niedere Alkylgruppen wie z.B. C1- bis C7-Alkylgruppen, besonders bevorzugt C1- bis C4-Alkylgruppen.

Monomere, welche quaternäre Amingruppen aufweisen, sind ungesättigte, radikalisch polymerisierbare Verbindungen, welche mindestens eine quaternäre Amingruppe tragen, insbesondere ammoniumsubstituierte Vinylmonomere oder quaternisierte Derivate von Carboxyvinylmonomeren wie z.B. quaternisierte Acrylamide oder Methacrylamide. Beispiele hierfür sind Acrylamidoalkyltrialkylammoniumhalogenide oder Methacrylamidoalkyltrialkylammoniumhalogenide, Trialkylmethacryloxyalkylammoniumhalogenide, Trialkylacryloxyalkylammoniumhalogenide, Dialkyldiallylammoniumhalogenide oder quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Bevorzugt sind Acrylamidopropyltrimethylammoniumchlorid und Methacrylamidopropyltrimethylammoniumchlorid.

Das kationische Polymer kann gegebenenfalls mit neutralen Comonomeren polymerisiert sein, die weder kationische noch kationisierbare Gruppen enthalten. Derartige neutrale Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Weitere kationische Polymere sind z.B. Polyvinylpyrrolidon/Dimethylaminoethylmethacrylat Copolymere, Copolymere aus Polyvinylpyrrolidon und Imidazoliminmethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcaprolactam, Vinylpyrrolidon/Methacrylamidopropyltrimethylammoniumchlorid Copolymere, Methacryloyl Ethyl Betain/Methacrylat Copolymere, Polymethacrylamidopropyl Trimonium Chlorid, Polyquaternium-2, Polyquaternium-6, Polyquaternium-7, Polyquaternium-22, Polyquaternium-27, Polyquaternium-39 sowie Polymere mit Siloxaneinheiten, z.B. Polyquaternium-41 oder Polyquaternium-42.

Ein erfindungsgemäßes Haarbehandlungsmittel enthält mindestens eine haarkonditionierende Substanz, ausgewählt aus Fettalkoholen und mindestens eine haarkonditionierende Substanz, ausgewählt aus kationischen Tensiden. Geeignete Fettalkohole sind Alkanole mit 8 bis 22 C-Atomen, z.B. Myristyl-, Cetyl- oder Stearylalkohol oder deren Mischungen. Die Fettalkohole werden vorzugsweise in einer Menge von 0,5 bis 10 Gew.%, besonders bevorzugt von 1 bis 7,5 Gew.%, insbesondere von 1,5 bis 5 Gew.% eingesetzt.

Die kationischen Tenside werden vorzugsweise in einer Menge von 0,1 bis 10 Gew.%, besonders bevorzugt von 0,25 bis 7,5 Gew.%, insbesondere von 0,5 bis 5 Gew.% eingesetzt. Geeignete kationische Tenside sind Tenside, welche eine quaternäre Ammoniumgruppe enthalten. Geeignete kationische Tenside können durch die allgemeine Formel (I) dargestellt werden:

$$N^{(+)}R^1R^2R^3R^4 \ X^{(-)} \qquad (I)$$

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens einer der Reste R1 bis R4 mindestens 8 C-Atome aufweist und $X^{(-)}$ ein Anion darstellt, z.B. ein Halogen, Acetat, Phosphat, Nitrat oder Alkylsulfat, vorzugsweise ein Chlorid. Die aliphatischen Gruppen können zusätzlich zu den C-Atomen und den Wasserstoffatomen auch Querverbindungen oder andere Gruppen wie beispielsweise Hydroxygruppen oder weitere Aminogruppen enthalten. Beispiele für geeignete kationische Tenside sind die Chloride oder Bromide von Alkyldimethylbenzylammoniumsalzen, Alkyltrimethylammoniumsalze, z.B. Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, z.B. Lauryl- oder Cetylpyridiniumchlorid oder Alkylamidoethyltrimethylammoniumethersulfate. Besonders bevorzugt sind Cetyl- und Behenyltrimethylammoniumchlorid sowie C8- bis C20-Alkylbetainester Salze, z.B. Cetylbetainesterchlorid.

Ein erfindungsgemäßes Haarbehandlungsmittel enthält vorzugsweise mindestens eine Puffersubstanz bzw. ein Puffersystem, welches im Bereich von etwa pH 3 bis 4, insbesondere beim isoelektrischen Punkt des menschlichen Haares eine ausreichende pH-Pufferkapazität aufweist. Derartige Systeme sind dem Fachmann geläufig.

Das erfindungsgemäße Haarbehandlungsmittel in Form eines Aerosol-Haarschaumproduktes (Mousse) enthält mindestens eine übliche, hierfür bekannte schaumgebende Substanz. Das Mittel wird mit Hilfe von Treibgasen oder chemischen Treibmitteln verschäumt, als Schaum in das Haar eingearbeitet und nach einer zur Entfaltung der konditionierenden Wirkung ausreichenden Einwirkzeit (maximal ca. 3 bis 15 Minuten) ausgespült. Das erfindungsgemäße Schaumprodukt weist als zusätzliche Komponente eine Vorrichtung zum Verschäumen der Zusammensetzung auf. Hierfür kann ein handelsüblicher Aerosolschaumkopf verwendet werden.

Die schaumgebende Substanz ist vorzugsweise ausgewählt aus Tensiden, insbesondere nichtionischen Tensiden mit einem HLB-Wert von maximal 20, vorzugsweise von 5 bis 18. Das schaumbildende Tensid ist vorzugsweise in einer Menge von 0,01 bis 15, besonders bevorzugt von 0,05 bis 10 Gew.% enthalten. Bevorzugt sind dabei ethoxylierte Tenside, wobei die Anzahl der Ethylenoxid-Einheiten zwischen 1 bis 1000, bevorzugt zwischen 1 bis 300, besonders bevorzugt zwischen 1 und 15 liegt. Bevorzugt werden Fettsäureglyceridethoxylate, Fettalkoholethoxylate, Fettaminethoxylate, Fettsäurealkanolamidethoxylate und Fettsäureesterethoxylate mit jeweils 1 bis 50 EO-Einheiten. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarbehandlungsmittel geeignet. Unter den Fettsäureesterethoxylaten sind vor allem Diglyceridethoxylate zu nennen wie das von der ICI Surfactants unter dem Handelnamen Arlatone® G vertriebene, mit 25 EO-Einheiten ethoxylierte Rizinusöl mit dem INCI-Namen PEG-25 Hydrogenated Castor Oil, das von der BASF unter dem Handelnamen Cremophor® El vertriebene, mit 35 EO-Einheiten ethoxylierte Rizinusöl mit dem INCI-Namen PEG-35 Castor Oil, das von der BASF unter dem Handelsnamen Cremophor® RH 410 vertriebene, mit 40 EO-Einheiten ethoxylierte, hydrierte Rizinusöl mit dem INCI-Namen PEG-40 Hydrogenated Castor Oil, und die von der Firma Witco Surfactants unter dem Namen Rewoderm® LI vertriebenen Rohstoffe zu nennen. Bevorzugte nicht-ionische Tenside sind ethoxylierte Fettsäurezuckerester, insbesondere ethoxylierte Sorbitanfettsäureester, die als Polysorbate bekannt sind, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Henkel unter dem Handelsnamen Plantaren® oder Plantacare® oder von der Firma Seppic unter dem Handelsnamen Oramix® vertrieben werden.

Das Treibmittel ist in dem erfindungsgemäßen Aerosol-Schaumprodukt vorzugsweise in einer Menge von 1 bis 20, besonders bevorzugt von 2 bis 10 Gew.% enthalten. Als Treibmittel sind beispielsweise niedere Alkane, wie zum Beispiel n-Butan, i-Butan, Propan, Butan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet. Bevorzugt sind Propan/Butan-Gemische.

Die verschäumbare Zusammensetzung wird in einer druckfesten Aerosolverpackung abgefüllt, welche mit einem Aersolschaumkopf versehen wird. Als Verpackungsmaterial können die hierfür üblichen Materialien wie Aluminium oder Weissblech verwendet werden. Besonders bevorzugt werden Schaumdruckröhren aus einem druckfesten Kunststoff wie z.B. Polyethylenterephtalat (PET) eingesetzt.

Gegenstand der Erfindung ist weiterhin ein Verfahren zur oxidativen Färbung und Konditionierung von Haaren. Bei diesem Verfahren werden die Haare zunächst mit einem üblichen oxidativen Haarfärbemittel gefärbt. Anschließend oder gleichzeitig werden die Haare mit einem zweiten Haarbehandlungsmittel gemäß Anspruch 1 behandelt. Zwischen dem Färbeschritt und der nachfolgenden Behandlung mit dem erfindungsgemäßen Haarbehandlungsmittel wird das Haar vorzugsweise ausgespült. Wird das Mittel auf nasses oder feuchtes Haar aufgetragen, so wird nach einer Einwirkzeit von vorzugsweise ca. 3 bis 15 Minuten, die auch unter Wärmeeinwirkung erfolgen kann, ausgespült. Anschließend werden die Haare gegebenenfalls getrocknet.

Die nachfolgenden Beispiele sollen den Gegenstand der Erfindung näher erläutern.

**Beispiele**

**Färbemittel A:**

    Koleston® Perfect Nuance 4/6; Welloxon® 6%
    (Handelsprodukte der Firma Wella AG)

**Färbemittel B:**

Koleston® Perfect Nuance 6/45; Welloxon® 6%
(Handelsprodukte der Firma Wella AG)

**Zusammensetzung 1:**

| 2,5 g | Cetearylalkohol |
| 2,0 g | Cetyltrimethylammoniumchlorid |
| 0,1 g | Merquat® 550 L (Dimethyldiallylammoniumchlorid/Acrylamid Copolymer, 8%ig in Wasser) |
| ad 100 g | Wasser |

eingestellt auf pH 3,8

**Zusammensetzung 2:**

| 2,5 g | Cetearylalkohol |
| 2,0 g | Cetyltrimethylammoniumchlorid |
| ad 100 g | Wasser |

eingestellt auf pH 3,8

**Zusammensetzung 3:**

| 2,5 g | Cetearylalkohol |
| 2,0 g | Cetyltrimethylammoniumchlorid |
| 0,1 g | Merquat® 550 L (Dimethyldiallylammoniumchlorid/Acrylamid Copolymer, 8%ig in Wasser) |
| ad 100 g | Wasser |

eingestellt auf pH 4,5

**Vergleichsversuche**

Haarvorbereitung:

Gebleichte Naturhaarsträhnen wurden mit einer Laurylethersulfat enthaltenden Lösung gewaschen und mit den Haarfärbemitteln A bzw. B oxidativ gefärbt. Danach wurden die Haarsträhnen mit einem der Nachbehandlungsmittel der Zusammensetzungen 1, 2, oder 3 behandelt und getrocknet. Pro zu untersuchender Zusammensetzung wurden Messungen an drei Strähnen durchgeführt. Es folgte jeweils 10-maliges Waschen pro Strähne mit Laurylethersulfat enthaltenden Haarreinigungsmitteln. Als Referenz zur Ermittlung der Lab-Werte wurde eine Strähne verwendet, welche nur gefärbt und einmal gewaschen, aber nicht mit einem Nachbehandlungsmittel behandelt wurde.

Vergleichsmethode:

Die Stabilität der Färbung der Haarsträhnen wurde durch Messung der im L*a*b*-Werte mit Hilfe des Spektrophotometers CM-508i (Minolta) ermittelt. Gemessen wird am Ansatz, in der Mitte und an der Spitze der Strähne. Aus den drei Messwerten wird der Mittelwert bestimmt. Die folgenden Angaben beziehen sich auf die Farbbeschreibung im L*, a*, b*-System. Die Differenz der L*, a* und b*-Werte zu denjenigen der Referenzsträhne ergibt die ΔL, Δa und Δb Werte. Als Maß für die Veränderung der Färbung durch Auswaschen der Farbe dient die Größe dE. Diese Größe wird berechnet nach:

$$dE = (\Delta L^2 + \Delta a^2 + \Delta b^2)^{1/2}$$

Ergebnis:

| | DE | | | DE |
|---|---|---|---|---|
| Zusammensetzung 1 Färbemittel A | 9,86 | | Zusammensetzung 1 Färbemittel B | 10,15 |
| Zusammensetzung 2 Färbemittel A | 15,59 | | Zusammensetzung 2 Färbemittel B | 12,74 |
| Zusammensetzung 3 Färbemittel A | 12,59 | | Zusammensetzung 3 Färbemittel B | 12,14 |

Die erfindungsgemäße Zusammensetzung 1 führt zu einem dE-Wert von 9,86 bei der Färbung mit Färbemittel A und zu einem dE-Wert von 10,15 bei der Färbung mit Färbemittel B. Die nicht erfindungsgemäßen Zusammensetzungen 2 (ohne kationisches Polymer) und 3 (pH 4,5) führten zu schlechteren Farbstabilitäten und höheren dE-Werten von 15,59 bzw. 12,59 für Färbungen mit Färbemittel A und 12,74 bzw. 12,14 für Färbungen mit Färbemittel B.
Die Farbverschiebungen lassen sich auch visuell an den Haarsträhnen erkennen.

**Beispiel 1**

| 2,5 g | Cetearylalkohol |
|---|---|
| 2,0 g | Behenyltrimethylammoniumchlorid |
| 0,1 g | Merquat® 550 L (Dimethyldiallylammoniumchlorid/Acrylamid Copolymer, 8%ig in Wasser) |
| ad 100 g | Wasser |

eingestellt auf pH 3,8
94 Gew.% der Zusammensetzung wurden mit 6 Gew.% Propan/Butan-Treibgas in eine PET-Schaumdruckröhre abgefüllt.

**Beispiel 2**

| 2,5 g | Cetearylalkohol |
|---|---|
| 2,0 g | Cetylbetainesterchlorid |
| 0,1 g | Merquat® 550 L (Dimethyldiallylammoniumchlorid/Acrylamid Copolymer, 8%ig in Wasser) |
| ad 100 g | Wasser |

eingestellt auf pH 3,8
94 Gew.% der Zusammensetzung wurden mit 6 Gew.% Propan/Butan-Treibgas in eine PET-Schaumdruckröhre abgefüllt.

**Patentansprüche**

1. Schaumbildendes Aerosol-Produkt bestehend aus (a) einer druckfesten Verpackung, (b) einem Schaumkopf, (c) einer auf den isoelektrischen Punkt von menschlichen Haaren, d.h. auf einen pH-Wert im Bereich von 3,6 bis 3,8 eingestellten wässrigen Lösung enthaltend nur ein Polymer, welches kationische Gruppen aufweist und ausgewählt ist aus Homo- und Copolymeren aus ungesättigten, radikalisch polymerisierbaren Monomeren, welche mindestens eine basische Gruppe oder mindestens eine quaternäre Amingruppe tragen und (d) mindestens ein Treibmittel; und worin die Lösung zusätzlich mindestens einen haarpflegenden Stoff enthält, der ausgewählt ist aus Fettalkoholen; und worin die Lösung zusätzlich mindestens einen haarpflegenden Stoff enthält, der ausgewählt ist aus kationischen Tensiden; und worin das kationische Polymer ein Dimethyldiallylammoniumchlorid/Acrylamid Copolymer ist.

2.  Verfahren zur Konditionierung und oxidativen Färbung von Haaren, wobei die Haare mit einem oxidativen Haarfarbemittel, welches zusätzlich mindestens einen synthetischen, direktziehenden Farbstoff oder mindestens einen pflanzlichen Farbstoff enthalten kann, gefärbt werden, und wobei die Haare anschließend oder gleichzeitig mit einem zweiten Haarbehandlungsmittel gemäß dem Anspruch 1 behandelt werden.

## Claims

1.  Foaming aerosol product consisting of (a) a pressure-resistant packaging, (b) a foam head, (c) an aqueous solution adjusted to the isoelectric point of human hair, i.e., to a pH value in the range of 3.6 to 3.8, said solution containing only one polymer which has cationic groups and is selected from homopolymers and copolymers of unsaturated, radically polymerizable monomers which carry at least one basic group or at least one quaternary amine group, and (d) at least one propellant; and wherein the solution additionally contains at least one hair-care substance selected from fatty alcohols; and wherein the solution additionally contains at least one hair-care substance selected from cationic surfactants; and wherein the cationic polymer is a dimethyldiallylammonium chloride/acrylamide copolymer.

2.  Method for conditioning and oxidative coloration of hair, wherein the hair is dyed with an oxidative hair colorant which may additionally contain at least one synthetic, direct dye or at least one vegetable dye, and wherein the hair is subsequently or simultaneously treated with a second hair treatment agent according to claim 1.

## Revendications

1.  Produit aérosol formant de la mousse, constitué par (a) un emballage résistant à la pression, (b) une tête mousseuse, (c) une solution aqueuse réglée au point isoélectrique des cheveux humains, c'est-à-dire à une valeur de pH dans la plage de 3,6 à 3,8, ne contenant qu'un seul polymère qui présente des groupes cationiques et qui est choisi parmi les homopolymères et les copolymères de monomères insaturés, polymérisables par voie radicalaire, qui portent au moins un groupe basique ou au moins un groupe amine quaternaire et (d) au moins un agent propulseur ; et la solution contenant en outre au moins une substance de soin des cheveux qui est choisie parmi les alcools gras; et la solution contenant en outre au moins une substance de soin des cheveux qui est choisie parmi les tensioactifs cationiques ; et le polymère cationique étant un copolymère de chlorure de diméthyldiallylammonium/acrylamide.

2.  Procédé pour le conditionnement et la teinture par oxydation de cheveux, les cheveux étant teintés par un colorant par oxydation des cheveux qui peut en outre contenir au moins un colorant synthétique montant directement sur les fibres ou au moins un colorant végétal et les cheveux étant traités ensuite ou simultanément par un deuxième agent de traitement des cheveux selon la revendication 1.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19735865

**In der Beschreibung aufgeführte Nicht-Patentliteratur**

- **C.R.ROBBINS.** Chemical and Physical Behavior of Human Hair. 204-206, 267